# EUROPEAN PATENT APPLICATION

(11) **EP 4 725 555 A2**
(43) Date of publication of application: **15.04.2026**
(21) Application number: 26160718.8
(22) Date of filing: 12.10.2022
(51) Int. Cl.: A61P 35/00

(54) **SINGLE-DOMAIN 4-1BB ANTIBODIES**

(30) Priority: 12.10.2021 WO PCT/CN2021/123368
(62) Divisional of application: 22880357.3
(71) Applicant: Lepu Biopharma Co., Ltd., Shanghai 201112 (CN)
(72) Inventor: GONG, Wenci, Shanghai 201203 (CN); YANG, Liu, Shanghai 201203 (CN); GAO, Shan, Shanghai 201203 (CN); YANG, Yuanyuan, Shanghai 201203 (CN); FANG, Lei, Shanghai 201203 (CN)
(74) Representative: Gleiss Große Schrell und Partner mbB

(57) **Abstract**

Provided are single domain anti-4-IBB antibodies and polypeptides, such as bispecific antibodies and chimeric antigen receptors, that include these single domain antibodies. These antibodies, including their humanized counterparts, exhibited superior activities and are suitable for use in various bispecific antibody formats. Methods of using the antibodies or polypeptides for treating and diagnosing diseases such as cancer are also provided.

## Description

### BACKGROUND

A single-domain antibody (sdAb), also known as a nanobody, is an antibody fragment consisting of a single monomeric variable antibody domain. Nanobodies produced from camelids and certain other animals are also referred to as VHH fragments. Like a whole antibody, a nanobody is able to bind selectively to a specific antigen. With a molecular weight of only 12-15 kDa, single domain antibodies are much smaller than common antibodies (150-160 kDa). Single domain antibodies, given their small sizes and one-chain nature, can be particularly suitable for inclusion as a fragment in other proteins, such chimeric antigen receptors (CAR) and bispecific antibodies.

4-1BB (CD137, tumor necrosis factor receptor superfamily 9) is a member of TNF-receptor superfamily (TNFRSF) and is a costimulatory molecule which is expressed following the activation of immune cells, both innate and adaptive immune cells. 4-1BB plays an important role in modulating the activity of various immune cells. 4-1BB agonists enhance immune cell proliferation, survival, secretion of cytokines and cytolytic activity CD8 T cells. Many other studies showed that activation of 4-1BB enhances immune response to eliminate tumors in mice. Therefore, it was suggested that 4-1BB is a promising target molecule in cancer immunology.

### SUMMARY

Provided are nanobodies specific to the human 4-1BB protein, and bi- or multispecific antibodies incorporating such a nanobody. In some embodiments, the nanobodies of the present disclosure are "non-agonist," which do not activate 4-1BB signaling on their own. When combined with an anti-Claudin 18.2 antibody, however, the ensuing bispecific antibody has potent activity in activating 4-1BB signaling in the presence of Claudin 18.2 positive cells.

4-1BB signaling activation is the expected mechanism for agonist antibodies, such as utomilumab (PF-05082566) and urelumab (BMS-663513). The anti-4-1BB nanobodies disclosed herein, however, do not require such an activity. Actually, it is preferred that the anti-4-1BB portion of a bispecific antibody is not capable of independently activating 4-1BB in the absence of binding to a tumor associated antigen (TAA) that the bispecific antibody also targets, as such bispecific antibodies would have reduced off-tumor side effects.

Compared to the known anti-4-1BB agonist antibodies which are commonly associated with dose-limiting on-target toxicities, the antibodies of the present disclosure are much safer. In a tissue, such as liver, wherein a corresponding TAA is not expressed, the bispecific antibodies of the present disclosure are not expected to trigger cytotoxic immune response as they cannot activate 4-1BB signaling. In a tumor tissue wherein the TAA is expressed and/or accessible, by contrast, these bispecific antibodies can initiate potent immune response to the tumor cells. Accordingly, unlike those anti-4-1BB antibodies currently being developed clinically which suffer on-target/inherent toxicities, the presently disclosed antibodies can be potent and safe at the same time in treating cancer.

Accordingly, in one embodiment of the present disclosure, provided is a single domain antibody or a polypeptide comprising the single domain antibody, wherein the single domain antibody has binding specificity to the human 4-1BB protein and comprises a complementarity determining region 1 (CDR1), a CDR2 and a CDR3, wherein the CDR1 comprises the amino acid sequence of SEQ ID NO:6, the CDR2 comprises the amino acid sequence of SEQ ID NO:7, 39, or 40, and the CDR3 comprises the amino acid sequence of SEQ ID NO:8.

In another embodiment, provided is a single domain antibody or a polypeptide comprising the single domain antibody, wherein the single domain antibody has binding specificity to the human 4-1BB protein and comprises a complementarity determining region 1 (CDR1), a CDR2 and a CDR3, wherein the CDR1 comprises the amino acid sequence of SEQ ID NO:9, the CDR2 comprises the amino acid sequence of SEQ ID NO:10, 41 or 42, and the CDR3 comprises the amino acid sequence of SEQ ID NO:11.

In another embodiment, provided is a single domain antibody or a polypeptide comprising the single domain antibody, wherein the single domain antibody has binding specificity to the human 4-1BB protein and comprises a complementarity determining region 1 (CDR1), a CDR2 and a CDR3, wherein the CDR1 comprises the amino acid sequence of SEQ ID NO:12, the CDR2 comprises the amino acid sequence of SEQ ID NO:13, 43 or 44, and the CDR3 comprises the amino acid sequence of SEQ ID NO:14.

In another embodiment, provided is a single domain antibody or a polypeptide comprising the single domain antibody, wherein the single domain antibody has binding specificity to the human 4-1BB protein and comprises a complementarity determining region 1 (CDR1), a CDR2 and a CDR3, wherein the CDR1, CDR2 and CDR3 comprise the amino acid sequences of SEQ ID NO:15-17, respectively.

In another embodiment, provided is a single domain antibody or a polypeptide comprising the single domain antibody, wherein the single domain antibody has binding specificity to the human 4-1BB protein and comprises a complementarity determining region 1 (CDR1), a CDR2 and a CDR3, wherein the CDR1, CDR2 and CDR3 comprise the amino acid sequences of SEQ ID NO:18-20, respectively.

In another embodiment, provided is a single domain antibody or a polypeptide comprising the single domain antibody, wherein the single domain antibody has binding specificity to the human 4-1BB protein and comprises a complementarity determining region 1 (CDR1), a CDR2 and a CDR3, wherein the CDR1, CDR2 and CDR3 comprise the amino acid sequences of the CDR1, CDR2 and CDR3 of anyone of SEQ ID NO:1-5, respectively.

Bispecific/multispecific antibodies are also provided that incorporate one or more units of the nanobodies. In some embodiments, the second specificity is to a tumor antigen, such as Claudin 18.2. Other example tumor antigens are also disclosed herein. In some embodiments, a chimeric antigen receptor (CAR) is provided that includes a nanobody of the present disclosure.

Also provided is a composition comprising the antibody or the polypeptide and a pharmaceutically acceptable carrier. Still also provided is one or more polynucleotide encoding the antibody or the polypeptide, an isolated cell comprising one or more polynucleotide encoding the antibody or fragment thereof.

Treatment methods and uses are also provided. In one embodiment, a method of treating cancer in a patient in need thereof is provided, comprising administering to the patient an effective amount of the antibody or the polypeptide of the present disclosure. In some embodiments, the cancer is a solid tumor. In some embodiments, the cancer is selected from the group consisting of bladder cancer, liver cancer, colon cancer, rectal cancer, endometrial cancer, leukemia, lymphoma, pancreatic cancer, small cell lung cancer, non-small cell lung cancer, breast cancer, urethral cancer, head and neck cancer, gastrointestinal cancer, stomach cancer, oesophageal cancer, ovarian cancer, renal cancer, melanoma, prostate cancer and thyroid cancer. In some embodiments, the method further comprises administering to the patient a second cancer therapeutic agent.

### BRIEF DESCRIPTION OF THE DRAWINGS

**FIG. 1** shows the ELISA results testing the binding activity of the anti-4-1BB antibodies to the human 4-1BB antigen, cyno 4-1BB antigen and mouse 4-1BB antigen.
**FIG. 2** shows cell-based binding to human 4-1BB protein.
**FIG.3** shows the full kinetic activity of tested anti-4-1BB antibodies measured by *Biocore.*
**FIG. 4** presents ELISA binding charts to show that the tested antibodies do not react with human OX40 or human CD40.
**FIG. 5** shows that unlike reference anti-4-1BB antibody Urelumab, the tested antibodies did not active 4-1BB signaling.
**FIG. 6** shows that the tested bispecific antibodies activated 4-1BB signaling only in Claudin 18.2 positive CHO cells.
**FIG. 7** shows that the tested bispecific antibodies activated 4-1BB signaling in PBMC cells only when in the presence of CHO cells over-expressing Claudin 18.2.
**FIG. 8** shows that the bispecific antibodies incorporating the humanized B31 antibody had NFκB activation activities comparable to the chimeric B31 antibody.
**FIG. 9** shows that the bispecific antibodies incorporating the humanized B31 antibody had IL-2 secretion induction activities comparable to the chimeric B31 antibody.
**FIG. 10** shows the *in vivo* tumor growth inhibition activities of the tested antibodies.

### DETAILED DESCRIPTION

### Definitions

It is to be noted that the term "a" or "an" entity refers to one or more of that entity; for example, "an antibody," is understood to represent one or more antibodies. As such, the terms "a" (or "an"), "one or more," and "at least one" can be used interchangeably herein.

A polynucleotide or polynucleotide region (or a polypeptide or polypeptide region) has a certain percentage (for example, 60 %, 65 %, 70 %, 75 %, 80 %, 85 %, 90 %, 95 %, 98 % or 99 %) of "sequence identity" to another sequence means that, when aligned, that percentage of bases (or amino acids) are the same in comparing the two sequences. This alignment and the percent homology or sequence identity can be determined using software programs known in the art, for example those described in Ausubel et al. eds. (2007) Current Protocols in Molecular Biology. Preferably, default parameters are used for alignment. One alignment program is BLAST, using default parameters. In particular, programs are BLASTN and BLASTP, using the following default parameters: Genetic code = standard; filter = none; strand = both; cutoff = 60; expect = 10; Matrix = BLOSUM62; Descriptions = 50 sequences; sort by = HIGH SCORE; Databases = non-redundant, GenBank + EMBL + DDBJ + PDB + GenBank CDS translations + SwissProtein + SPupdate + PIR. Biologically equivalent polynucleotides are those having the above-noted specified percent homology and encoding a polypeptide having the same or similar biological activity.

The term "an equivalent nucleic acid or polynucleotide" refers to a nucleic acid having a nucleotide sequence having a certain degree of homology, or sequence identity, with the nucleotide sequence of the nucleic acid or complement thereof. A homolog of a double stranded nucleic acid is intended to include nucleic acids having a nucleotide sequence which has a certain degree of homology with or with the complement thereof. In one aspect, homologs of nucleic acids are capable of hybridizing to the nucleic acid or complement thereof. Likewise, "an equivalent polypeptide" refers to a polypeptide having a certain degree of homology, or sequence identity, with the amino acid sequence of a reference polypeptide. In some aspects, the sequence identity is at least about 70%, 75%, 80%, 85%, 90%, 95%, 98%, or 99%. In some aspects, the equivalent polypeptide or polynucleotide has one, two, three, four or five addition, deletion, substitution and their combinations thereof as compared to the reference polypeptide or polynucleotide. In some aspects, the equivalent sequence retains the activity (*e.g.,* epitope-binding) or structure (*e.g.,* salt-bridge) of the reference sequence.

As used herein, an "antibody" or "antigen-binding polypeptide" refers to a polypeptide or a polypeptide complex that specifically recognizes and binds to an antigen. An antibody can be a whole antibody and any antigen binding fragment or a single chain thereof. Thus the term "antibody" includes any protein or peptide containing molecule that comprises at least a portion of an immunoglobulin molecule having biological activity of binding to the antigen. Examples of such include, but are not limited to a complementarity determining region (CDR) of a heavy or light chain or a ligand binding portion thereof, a heavy chain or light chain variable region, a heavy chain or light chain constant region, a framework (FR) region, or any portion thereof, or at least one portion of a binding protein.

The terms "antibody fragment" or "antigen-binding fragment", as used herein, is a portion of an antibody such as F(ab')₂, F(ab)₂, Fab', Fab, Fv, scFv and the like. Regardless of structure, an antibody fragment binds with the same antigen that is recognized by the intact antibody. The term "antibody fragment" includes aptamers, spiegelmers, and diabodies. The term "antibody fragment" also includes any synthetic or genetically engineered protein that acts like an antibody by binding to a specific antigen to form a complex.

A "single-chain variable fragment" or "scFv" refers to a fusion protein of the variable regions of the heavy (V_{H}) and light chains (V_{L}) of immunoglobulins. In some aspects, the regions are connected with a short linker peptide of ten to about 25 amino acids. The linker can be rich in glycine for flexibility, as well as serine or threonine for solubility, and can either connect the N-terminus of the V_{H} with the C-terminus of the V_{L}, or vice versa. This protein retains the specificity of the original immunoglobulin, despite removal of the constant regions and the introduction of the linker. ScFv molecules are known in the art and are described, *e.g*., in US patent 5,892,019.

The term antibody encompasses various broad classes of polypeptides that can be distinguished biochemically. Those skilled in the art will appreciate that heavy chains are classified as gamma, mu, alpha, delta, or epsilon (y, µ, α, δ, ε) with some subclasses among them (*e.g.,* γ1- γ4). It is the nature of this chain that determines the "class" of the antibody as IgG, IgM, IgA IgG, or IgE, respectively. The immunoglobulin subclasses (isotypes) *e.g*., IgG₁, IgG₂, IgG₃, IgG₄, IgG₅, etc. are well characterized and are known to confer functional specialization. Modified versions of each of these classes and isotypes are readily discernable to the skilled artisan in view of the instant disclosure and, accordingly, are within the scope of the instant disclosure. All immunoglobulin classes are clearly within the scope of the present disclosure, the following discussion will generally be directed to the IgG class of immunoglobulin molecules. With regard to IgG, a standard immunoglobulin molecule comprises two identical light chain polypeptides of molecular weight approximately 23,000 Daltons, and two identical heavy chain polypeptides of molecular weight 53,000-70,000. The four chains are typically joined by disulfide bonds in a "Y" configuration wherein the light chains bracket the heavy chains starting at the mouth of the "Y" and continuing through the variable region.

Antibodies, antigen-binding polypeptides, variants, or derivatives thereof of the disclosure include, but are not limited to, polyclonal, monoclonal, multispecific, human, humanized, primatized, or chimeric antibodies, single chain antibodies, epitope-binding fragments, *e.g*., Fab, Fab' and F(ab')₂, Fd, Fvs, single-chain Fvs (scFv), single-chain antibodies, disulfide-linked Fvs (sdFv), fragments comprising either a VK or VH domain, fragments produced by a Fab expression library, and anti- idiotypic (anti-Id) antibodies (including, *e.g*., anti-Id antibodies to LIGHT antibodies disclosed herein). Immunoglobulin or antibody molecules of the disclosure can be of any type (*e.g.,* IgG, IgE, IgM, IgD, IgA, and IgY), class (e.g., IgG1, IgG2, IgG3, IgG4, IgAl and IgA2) or subclass of immunoglobulin molecule.

By "specifically binds" or "has specificity to," it is generally meant that an antibody binds to an epitope via its antigen-binding domain, and that the binding entails some complementarity between the antigen-binding domain and the epitope. According to this definition, an antibody is said to "specifically bind" to an epitope when it binds to that epitope, via its antigen-binding domain more readily than it would bind to a random, unrelated epitope. The term "specificity" is used herein to qualify the relative affinity by which a certain antibody binds to a certain epitope. For example, antibody "A" may be deemed to have a higher specificity for a given epitope than antibody "B," or antibody "A" may be said to bind to epitope "C" with a higher specificity than it has for related epitope "D."

As used herein, the terms "treat" or "treatment" refer to both therapeutic treatment and prophylactic or preventative measures, wherein the object is to prevent or slow down (lessen) an undesired physiological change or disorder, such as the progression of cancer. Beneficial or desired clinical results include, but are not limited to, alleviation of symptoms, diminishment of extent of disease, stabilized (*i.e.,* not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the condition or disorder is to be prevented.

By "subject" or "individual" or "animal" or "patient" or "mammal," is meant any subject, particularly a mammalian subject, for whom diagnosis, prognosis, or therapy is desired. Mammalian subjects include humans, domestic animals, farm animals, and zoo, sport, or pet animals such as dogs, cats, guinea pigs, rabbits, rats, mice, horses, cattle, cows, and so on.

As used herein, phrases such as "to a patient in need of treatment" or "a subject in need of treatment" includes subjects, such as mammalian subjects, that would benefit from administration of an antibody or composition of the present disclosure used, *e.g.,* for detection, for a diagnostic procedure and/or for treatment.

### Single Domain Anti-4-1BB Antibodies

The present disclosure provides single chain anti-4-1BB antibodies with high affinity to the human 4-1BB protein. In some embodiments, the provided nanobodies are not capable of activating 4-1BB signaling merely by such binding, and thus are referred to as "non-agonist" anti-4-1BB antibodies. Such non-agonist anti-4-1BB nanobodies are particularly suitable for preparing bispecific antibodies.

4-1BB signaling activation is the expected mechanism for agonist antibodies, such as utomilumab (PF-05082566) and urelumab (BMS-663513). The anti-4-1BB nanobodies disclosed herein, however, do not require have such an activity. Actually, it is preferred that the anti-4-1BB portion of a bispecific antibody is not capable of independently activating 4-1BB in the absence of CLDN18.2 binding, as such bispecific antibodies would have reduced off-target side effects.

Compared to known anti-4-1BB agonist antibodies, such as utomilumab (PF-05082566) and urelumab (BMS-663513), which are commonly associated with dose-limiting on-target toxicities, the bispecific antibodies of the present disclosure are much safer. In a tissue wherein CLDN18.2 is not expressed, the bispecific antibodies of the present disclosure are not expected to trigger cytotoxic immune response as they cannot activate 4-1BB signaling. In a tumor tissue wherein CLDN18.2 is expressed and/or accessible, by contrast, these bispecific antibodies can initiate potent immune response to the tumor cells. Accordingly, unlike those anti-4-1BB antibodies currently being developed clinically which suffer on-target/inherent toxicities, the presently disclosed antibodies can be potent and safe at the same time in treating cancer.

Accordingly, in one embodiment of the present disclosure, provided are single domain antibodies and polypeptides that include such a single domain antibody. In one embodiment of the present disclosure, provided is a single domain antibody or a polypeptide comprising the single domain antibody, wherein the single domain antibody includes a CDR1, a CDR2 and a CDR3, which respectively have the CDR1, CDR2 and CDR3 sequences of antibody B31 (SEQ ID NO:1). In some embodiments, the CDR1 includes the amino acid sequence of SEQ ID NO:6, the CDR2 includes the amino acid sequence of SEQ ID NO:7, 39 or 40, and the CDR3 includes the amino acid sequence of SEQ ID NO:8. In some embodiments, the CDR1, CDR2 and CDR3 include the amino acid sequences of SEQ ID NO:6-8, respectively. In some embodiments, the CDR1, CDR2 and CDR3 include the amino acid sequences of SEQ ID NO:6, 39 and 8, respectively. In some embodiments, the CDR1, CDR2 and CDR3 include the amino acid sequences of SEQ ID NO:6, 40 and 8, respectively.

Humanized antibodies are also provided, such as those provided in SEQ ID NO:21-25 for antibody B31. In some embodiments, the humanized antibodies include back mutations selected from the group consisting of 23A, 37Y, 40P, 41Q, 44Q, 45R, 49A, 74N, 78M, 82(82A)D, and 94A, according to Kabat numbering. In some embodiments, the humanized antibodies include back mutations 37Y, 44Q, 45R and 94A. In some embodiments, the humanized antibodies include back mutations 23A, 37Y, 44Q, 45R, 49A, 74N and 94A. In some embodiments, the humanized antibodies include back mutations 23A, 37Y, 44Q, 45R, 49A, 78M, 82(82A)D, and 94A. In some embodiments, the humanized antibodies include back mutations 23A, 37Y, 40P, 41Q, 44Q, 45R, 49A, 74N, 82(82A)D, and 94A. In some embodiments, the humanized antibodies include back mutations 37Y, 44Q, 45R, and 49A.

In some embodiments, the antibody includes the amino acid sequence of SEQ ID NO:1. In some embodiments, the antibody includes a humanized sequence of SEQ ID NO:21. In some embodiments, the antibody includes a humanized sequence of SEQ ID NO:22. In some embodiments, the antibody includes a humanized sequence of SEQ ID NO:23. In some embodiments, the antibody includes a humanized sequence of SEQ ID NO:24. In some embodiments, the antibody includes a humanized sequence of SEQ ID NO:25. In some embodiments, the antibody includes the recited CDR1, CDR2 and CDR3 and has at least 80%, 85%, 90%, 95%, 98%, or 99% sequence identity to any one of SEQ ID NO:1 and 21-25.

In another embodiment, provided is a single domain antibody or a polypeptide comprising the single domain antibody, wherein the single domain antibody includes a CDR1, a CDR2 and a CDR3, which respectively have the CDR1, CDR2 and CDR3 sequences of antibody B16 (SEQ ID NO:2). In some embodiments, the CDR1 includes the amino acid sequence of SEQ ID NO:9, the CDR2 includes the amino acid sequence of SEQ ID NO:10, 41 or 42, and the CDR3 includes the amino acid sequence of SEQ ID NO:11. In some embodiments, the CDR1, CDR2 and CDR3 include the amino acid sequences of SEQ ID NO:9-11, respectively. In some embodiments, the CDR1, CDR2 and CDR3 include the amino acid sequences of SEQ ID NO:9, 41 and 11, respectively. In some embodiments, the CDR1, CDR2 and CDR3 include the amino acid sequences of SEQ ID NO:9, 42 and 11, respectively.

Humanized antibodies are also provided, such as those provided in SEQ ID NO:31-34 for antibody B16. In some embodiments, the humanized antibodies include back mutations selected from the group consisting of 29L, 30D, 37F, 44E, 45R, 47G, 74A, 84P, and 94T, according to Kabat numbering. In some embodiments, the humanized antibodies include back mutations selected from the group consisting of 37F, 44E, 45R, 47G, and 94T, according to Kabat numbering. In some embodiments, the humanized antibodies include back mutations selected from the group consisting of 29L, 30D, 37F, 44E, 45R, 47G, and 94T, according to Kabat numbering. In some embodiments, the humanized antibodies include back mutations selected from the group consisting of 29L, 30D, 37F, 44E, 45R, 47G, 74A, 84P, and 94T, according to Kabat numbering.

In some embodiments, the antibody includes the amino acid sequence of SEQ ID NO:2. In some embodiments, the antibody includes a humanized sequence of SEQ ID NO:31. In some embodiments, the antibody includes a humanized sequence of SEQ ID NO:32. In some embodiments, the antibody includes a humanized sequence of SEQ ID NO:33. In some embodiments, the antibody includes a humanized sequence of SEQ ID NO:34. In some embodiments, the antibody includes the recited CDR1, CDR2 and CDR3 and has at least 80%, 85%, 90%, 95%, 98%, or 99% sequence identity to any one of SEQ ID NO:2 and 31-34.

In another embodiment, provided is a single domain antibody or a polypeptide comprising the single domain antibody, wherein the single domain antibody includes a CDR1, a CDR2 and a CDR3, which respectively have the CDR1, CDR2 and CDR3 sequences of antibody B125 (SEQ ID NO:3). In some embodiments, the CDR1 includes the amino acid sequence of SEQ ID NO: 12, the CDR2 includes the amino acid sequence of SEQ ID NO:13, 43 or 44, and the CDR3 includes the amino acid sequence of SEQ ID NO: 14. In some embodiments, the CDR1, CDR2 and CDR3 include the amino acid sequences of SEQ ID NO:12-14, respectively. In some embodiments, the CDR1, CDR2 and CDR3 include the amino acid sequences of SEQ ID NO:12, 43 and 14, respectively. In some embodiments, the CDR1, CDR2 and CDR3 include the amino acid sequences of SEQ ID NO:12, 44 and 14, respectively.

Humanized antibodies are also provided, such as those provided in SEQ ID NO:26-30 for antibody B125. In some embodiments, the humanized antibodies include back mutations selected from the group consisting of 37F, 43K, 44E, 45R, 47G, 74A, 84P, and 94T, according to Kabat numbering. In some embodiments, the humanized antibodies include back mutations selected from the group consisting of 37F, 44E, 45R, 47G, and 94T, according to Kabat numbering. In some embodiments, the humanized antibodies include back mutations selected from the group consisting of 37F, 43K, 44E, 45R, 47G, and 74A, according to Kabat numbering. In some embodiments, the humanized antibodies include back mutations selected from the group consisting of 37F, 43K, 44E, 45R, 47G, 74A, and 94T, according to Kabat numbering. In some embodiments, the humanized antibodies include back mutations selected from the group consisting of 37F, 43K, 44E, 45R, 47G, 74A, 84P, and 94T, according to Kabat numbering.

In some embodiments, the antibody includes the amino acid sequence of SEQ ID NO:3. In some embodiments, the antibody includes a humanized sequence of SEQ ID NO:26. In some embodiments, the antibody includes a humanized sequence of SEQ ID NO:27. In some embodiments, the antibody includes a humanized sequence of SEQ ID NO:28. In some embodiments, the antibody includes a humanized sequence of SEQ ID NO:29. In some embodiments, the antibody includes a humanized sequence of SEQ ID NO:30. In some embodiments, the antibody includes the recited CDR1, CDR2 and CDR3 and has at least 80%, 85%, 90%, 95%, 98%, or 99% sequence identity to any one of SEQ ID NO:3 and 26-30.

In another embodiment, provided is a single domain antibody or a polypeptide comprising the single domain antibody, wherein the single domain antibody includes a CDR1, a CDR2 and a CDR3, which respectively have the CDR1, CDR2 and CDR3 sequences of antibody B164 (SEQ ID NO:4). In some embodiments, the CDR1, CDR2 and CDR3 include the amino acid sequences of SEQ ID NO: 15-17, respectively. In some embodiments, the antibody includes the recited CDR1, CDR2 and CDR3 and has at least 80%, 85%, 90%, 95%, 98%, or 99% sequence identity to SEQ ID NO:4.

In another embodiment, provided is a single domain antibody or a polypeptide comprising the single domain antibody, wherein the single domain antibody includes a CDR1, a CDR2 and a CDR3, which respectively have the CDR1, CDR2 and CDR3 sequences of antibody B210 (SEQ ID NO:5). In some embodiments, the CDR1, CDR2 and CDR3 include the amino acid sequences of SEQ ID NO:18-20, respectively.

Humanized antibodies are also provided, such as those provided in SEQ ID NO:26-30 for antibody B210. In some embodiments, the humanized antibodies include back mutations selected from the group consisting of 27L, 29L, 37F, 44E, 45R, 47G, 89D, and 94T, according to Kabat numbering. In some embodiments, the humanized antibodies include back mutations selected from the group consisting of 37F, 44E, 45R, 47G, and 94T, according to Kabat numbering. In some embodiments, the humanized antibodies include back mutations selected from the group consisting of 27L, 37F, 44E, 45R, 47G, and 94T, according to Kabat numbering. In some embodiments, the humanized antibodies include back mutations selected from the group consisting of 27L, 29L, 37F, 44E, 45R, 47G, 89D, and 94T, according to Kabat numbering.

In some embodiments, the antibody includes the amino acid sequence of SEQ ID NO:5. In some embodiments, the antibody includes a humanized sequence of SEQ ID NO:35. In some embodiments, the antibody includes a humanized sequence of SEQ ID NO:36. In some embodiments, the antibody includes a humanized sequence of SEQ ID NO:37. In some embodiments, the antibody includes a humanized sequence of SEQ ID NO:38. In some embodiments, the antibody includes the recited CDR1, CDR2 and CDR3 and has at least 80%, 85%, 90%, 95%, 98%, or 99% sequence identity to any one of SEQ ID NO:5 and 35-38.

Also provided, in some embodiments, are anti-4-1BB antibodies and antigen binding fragments that compete with any of the antibodies disclosed herein in binding to human 4-1BB. Also provided, in some embodiments, are anti-4-1BB antibodies and antigen binding fragments that bind to the same epitope as any of the antibodies disclosed herein. Also provided, in some embodiments, are anti-4-1BB antibodies and antigen binding fragments that included the CDR1, CDR2, and CDR3 of the antibodies disclosed herein.

Also provided are compositions that include the antibody or the polypeptide and a pharmaceutically acceptable carrier.

It will also be understood by one of ordinary skill in the art that antibodies as disclosed herein may be modified such that they vary in amino acid sequence from the naturally occurring binding polypeptide from which they were derived. For example, a polypeptide or amino acid sequence derived from a designated protein may be similar, e.g., have a certain percent identity to the starting sequence, e.g., it may be 60%, 70%, 75%, 80%, 85%, 90%, 95%, 98%, or 99% identical to the starting sequence. In some embodiments, the modified antibody or fragment retains the designate CDR sequences.

In certain embodiments, the antibody comprises an amino acid sequence or one or more moieties not normally associated with an antibody. Exemplary modifications are described in more detail below. For example, an antibody of the disclosure may comprise a flexible linker sequence, or may be modified to add a functional moiety (e.g., PEG, a drug, a toxin, or a label).

Also provided are bispecific and multispecific antibodies that includes one, two, three or four units of the single domain anti-4-1BB antibody as disclosed herein, and one or more other specificities (not 4-1BB).

### Bispecific and Multispecific Antibodies, and Chimeric Antigen Receptors (CAR)

As provided, the anti-4-1BB antibodies disclosed here are particularly useful for preparing bispecific and multispecific antibodies, as well as chimeric antigen receptors (CAR). This is at least because of these antibodies' enhanced therapeutic index and their small sizes.

Accordingly, in one embodiment, provided is a bispecific antibody that includes an anti-4-1BB nanobody of the present disclosure, or an antigen-binding fragment thereof, and a second antibody or antigen-binding fragment having binding specificity to a target antigen that is not 4-1BB. In some embodiment, a third or fourth specificity is further included.

The target antigen that is not 4-1BB, in some embodiments, is a tumor antigen. An abundance of tumor antigens are known in the art and new tumor antigens can be readily identified by screening. Non-limiting examples of tumor antigens include ABL, ALK, B4GALNT1 , BAFF, BCL2, BRAF, BTK, CD19, CD20, CD30, CD38, CD52, CD73, Claudin 18.2, CTLA-4, EGFR, FOLR1, FLT3, HDAC, HER2, IDH2, IL-1β, IL-6, IL-6R, JAK1/2, JAK3, KIT, LAG-3, MEK, Nectin 4, ROR1, mTOR, PARP, PD-1, PDGFR, PDGFRα, PD-L1, PI3Kδ, PIGF, PTCH, RAF, RANKL, Smoothened, VEGF, VEGFR, and VEGFR2. Other examples are Her2, EpCAM, CD33, CD47, CD133, CEA, gpA33, Mucins, TAG-72, CIX, PSMA, GD2, GD3, GM2, Integrin, αVβ3, α5β1, ERBB2, ERBB3, MET, IGF1R, EPHA3, TRAILR1, TRAILR2, RANKL, FAP and Tenascin.

In some embodiments, the bispecific antibody has specificity to 4-1BB and Claudin 18.2.

Also provided, are chimeric antigen receptor (CAR) that includes a nanobody of the present disclosure. In the CAR, the nanobody can serve as the antigen recognition domain. In addition, in some embodiments, the CAR also includes an extracellular hinge region, a transmembrane domain, and an intracellular T-cell signaling domain.

The hinge, also called a spacer, is a small structural domain that sits between the antigen recognition region and the cell's outer membrane. A suitable hinge enhances the flexibility of the scFv receptor head, reducing the spatial constraints between the CAR and its target antigen. Example hinge sequences are based on membrane-proximal regions from immune molecules such as IgG, CD8, and CD28.

The transmembrane domain is a structural component, consisting of a hydrophobic alpha helix that spans the cell membrane. It anchors the CAR to the plasma membrane, bridging the extracellular hinge and antigen recognition domains with the intracellular signaling region. Typically, the transmembrane domain from a membrane-proximal component of the endodomain can be used, such as the CD28 transmembrane domain.

The intracellular T-cell signaling domain lies in the receptor's endodomain, inside the cell. After an antigen is bound to the external antigen recognition domain, CAR receptors cluster together and transmit an activation signal. Then the internal cytoplasmic end of the receptor perpetuates signaling inside the T cell. To mimic this process, CD3-zeta's cytoplasmic domain is commonly used as the main CAR endodomain component.

T cells also require co-stimulatory molecules in addition to CD3 signaling in order to persist after activation. In some embodiments, the endodomains of CAR receptor also includes one or more chimeric domains from co-stimulatory proteins, such as CD28, CD27, CD134 (OX40), and CD137 (4-1BB).

### Polynucleotides Encoding the Antibodies and Methods of Preparing the Antibodies

The present disclosure also provides isolated polynucleotides or nucleic acid molecules encoding the antibodies, variants or derivatives thereof of the disclosure. The polynucleotides of the present disclosure may encode the entire heavy and light chain variable regions of the antigen-binding polypeptides, variants or derivatives thereof on the same polynucleotide molecule or on separate polynucleotide molecules. Additionally, the polynucleotides of the present disclosure may encode portions of the heavy and light chain variable regions of the antigen-binding polypeptides, variants or derivatives thereof on the same polynucleotide molecule or on separate polynucleotide molecules.

Methods of making antibodies are well known in the art and described herein. In certain embodiments, both the variable and constant regions of the antigen-binding polypeptides of the present disclosure are fully human. Fully human antibodies can be made using techniques described in the art and as described herein. For example, fully human antibodies against a specific antigen can be prepared by administering the antigen to a transgenic animal which has been modified to produce such antibodies in response to antigenic challenge, but whose endogenous loci have been disabled. Exemplary techniques that can be used to make such antibodies are described in U.S. patents: 6,150,584; 6,458,592; 6,420,140 which are incorporated by reference in their entireties.

### Cancer Treatment

As described herein, the antibodies, bispecific antibodies, polypeptides, variants or derivatives of the present disclosure may be used in certain treatment and diagnostic methods.

The present disclosure is further directed to antibody-based therapies which involve administering the antibodies of the disclosure to a patient such as an animal, a mammal, and a human for treating one or more of the disorders or conditions described herein. Therapeutic compounds of the disclosure include, but are not limited to, antibodies of the disclosure (including variants and derivatives thereof as described herein) and nucleic acids or polynucleotides encoding antibodies of the disclosure (including variants and derivatives thereof as described herein).

In some embodiments, provided are methods for treating a cancer in a patient in need thereof. The method, in one embodiment, entails administering to the patient an effective amount of an antibody of the present disclosure. In some embodiments, at least one of the cancer cells (e.g., stromal cells) in the patient over-express a tumor antigen such as Claudin 18.2.

Cellular therapies, such as chimeric antigen receptor (CAR) T-cell or NK cell therapies, are also provided in the present disclosure. A suitable cell can be used, that is put in contact with an antibody or CAR of the present disclosure (or alternatively engineered to express an antibody or CAR of the present disclosure). Upon such contact or engineering, the cell can then be introduced to a cancer patient in need of a treatment. The cancer patient may have a cancer of any of the types as disclosed herein. The cell (e.g., T cell or NK cell) can be, for instance, a tumor-infiltrating T lymphocyte, a CD4+ T cell, a CD8+ T cell, or the combination thereof, without limitation.

In some embodiments, the cell was isolated from the cancer patient him- or her-self. In some embodiments, the cell was provided by a donor or from a cell bank. When the cell is isolated from the cancer patient, undesired immune reactions can be minimized.

Additional diseases or conditions associated with increased cell survival, that may be treated, prevented, diagnosed and/or prognosed with the antibodies or variants, or derivatives thereof of the disclosure include, but are not limited to, progression, and/or metastases of malignancies and related disorders such as leukemia (including acute leukemias (*e.g*., acute lymphocytic leukemia, acute myelocytic leukemia (including myeloblastic, promyelocytic, myelomonocytic, monocytic, and erythroleukemia)) and chronic leukemias (*e.g*., chronic myelocytic (granulocytic) leukemia and chronic lymphocytic leukemia)), polycythemia vera, lymphomas (*e.g*., Hodgkin's disease and non-Hodgkin's disease), multiple myeloma, Waldenstrom's macroglobulinemia, heavy chain disease, and solid tumors including, but not limited to, sarcomas and carcinomas such as fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyo sarcoma, colon carcinoma, pancreatic cancer, breast cancer, thyroid cancer, endometrial cancer, melanoma, prostate cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, menangioma, melanoma, neuroblastoma and retinoblastoma.

### Diagnostic Methods

Over-expression of 4-1BB is observed in certain tumor samples, and patients having 4-1BB-over-expressing cells are likely responsive to treatments with the anti-4-1BB antibodies of the present disclosure. Accordingly, the antibodies of the present disclosure can also be used for diagnostic and prognostic purposes.

A sample that preferably includes a cell can be obtained from a patient, which can be a cancer patient or a patient desiring diagnosis. The cell be a cell of a tumor tissue or a tumor block, a blood sample, a urine sample or any sample from the patient. Upon optional pretreatment of the sample, the sample can be incubated with an antibody of the present disclosure under conditions allowing the antibody to interact with a 4-1BB protein potentially present in the sample. Methods such as ELISA can be used, taking advantage of the anti-4-1BB antibody, to detect the presence of the 4-1BB protein in the sample.

Presence of the 4-1BB protein in the sample (optionally with the amount or concentration) can be used for diagnosis of cancer, as an indication that the patient is suitable for a treatment with the antibody, or as an indication that the patient has (or has not) responded to a cancer treatment. For a prognostic method, the detection can be done at once, twice or more, at certain stages, upon initiation of a cancer treatment to indicate the progress of the treatment.

### Compositions

The present disclosure also provides pharmaceutical compositions. Such compositions comprise an effective amount of an antibody, and an acceptable carrier. In some embodiments, the composition further includes a second anticancer agent (e.g., an immune checkpoint inhibitor).

In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. Further, a "pharmaceutically acceptable carrier" will generally be a non-toxic solid, semisolid or liquid filler, diluent, encapsulating material or formulation auxiliary of any type.

The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents such as acetates, citrates or phosphates. Antibacterial agents such as benzyl alcohol or methyl parabens; antioxidants such as ascorbic acid or sodium bisulfite; chelating agents such as ethylenediaminetetraacetic acid; and agents for the adjustment of tonicity such as sodium chloride or dextrose are also envisioned. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in Remington's Pharmaceutical Sciences by E. W. Martin, incorporated herein by reference. Such compositions will contain a therapeutically effective amount of the antigen-binding polypeptide, preferably in purified form, together with a suitable amount of carrier so as to provide the form for proper administration to the patient. The formulation should suit the mode of administration. The parental preparation can be enclosed in ampoules, disposable syringes or multiple dose vials made of glass or plastic.

In an embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lignocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

### EXAMPLES

### Example 1: Generation of VHH Antibodies against Human 4-1BB

This example describes generations of single domain (VHH) antibodies against the human 4-1BB protein.

*Immunization:* To generate VHH antibodies to human 4-1BB, two alpaca were immunized with human 4-1BB protein. Post 2 rounds of immunization, the serum of immunized alpaca was subject to the antibody titer evaluation by ELISA.

*Immunized library construction:* The phage library was constructed using phagemid vectors which consisted of the VHH gene fragments that were amplified from PBMC of 4-1BB immunized alpaca. The antibody format is VHH fragment in phage display library. Three immunized libraries were generated from PBMC of different alpaca at different immunization round. Every library size was more than 1× 10⁹ and the sequence diversity was analyzed as follows. For the 48 clones picked up from each library and further sequenced. Sequence showed enough diversity in CDRs for these three libraries.

*Phage Panning and Clone Selection:* 4-1BB protein was used as antigen for phage library panning.

*Phage library solution panning or solid phase panning against human 4-1BB:* The bound phages were eluted with Gly-Hcl. The resulting phage is out-put 1. The bound phages were incubated with SS320 cells and plated on 2YT plates for next round of panning screening. There was total 3-round of panning screening. The phage ELISA of output1, output2 and output3 showed enriched 4-1BB binders post three rounds of screening.

The single clones were pick up from output 2 and output 3 phages. The phage of these clones were subject to antigen binding ELISA. The clones showed good binding potency were selected for subsequent sequencing.

Five candidate sequences were cloned into pcDNA 3.4 vector and expressed in 293F cells. The monoclonal antibodies were purified from the culture supernatant by protein G. The purified antibodies were subjected to ELISA binding evaluation on 4-1BB-His protein.

The amino acid sequences of the single variable domains of B31, B16, B125, B164, B210 are listed in **Tables 1** below. The CDR sequences are summarized in **Table 1A-1E.** Some of the CDR sequences include dipeptides such as NG and DG which may be susceptible to post-translational modifications. Certain "de-risked" versions of these sequences are also provided in the table which are contemplated to retain the antibody activity while reducing such post-translational modification risks.

**Table 1. Variable domain sequences**

| **Name** | **Sequence** | **SEQ ID NO:** |
|---|---|---|
| B31 | QVQLVESGGGLVQAGGSLRLSCAASGDIFSIMFWYRQPQGKQREYVAHITSN GRTNYADSVTGRFTISRGNNDNTMYLQMDNLKPQDTAVYYCAADDATNTRTY WGQGTQVTVSS | 1 |
| B16 | QVQLVESGGGLVQPGGSLRLSCAASGFTLDYYAIGWFRQAPGKEREGVSCIS SSDGSTYYADSVKGRFTISRDNAKNTVYLQMNSLKPEDTAVYYCATDTTTRC PIWSYYVESDYWGQGTQVTVSS | 2 |
| B125 | QVQLVESGGGLVQPGGSLRLSCAASGFAFSDYAIGWFRQAPGKEREGVSCIS SSDGSTYYADSVKGRFTISRDNAKNTVYLQMNSLKPEDTAVYYCATDTTTRC PIWSYYVESDYWGQGTQVTVSS | 3 |
| B164 | EVQLVESGGGLVQAGGSLRLSCAASGSIFSYMFWYRQPQGKQREYVAHITSG GRTNYADSVTGRFTISRGNNDNTMYLQMNNLKPEDTAVYYCAADDAVYTDRT YWGQGTQVTVSS | 4 |
| B210 | EVQLVESGGGLVQPGGSLRLSCAASGLSLDYYAIGWFRQAPGKEREGVSCIS SSGRSTNYADSVKGRFTISRDNAKNTVYLQMNSLKPEDTADYYCATDTTTRC PIWSNYVESDYWGQGTQVTVSS | 5 |

**Table 1A. CDR sequences of B31**

| **Name** | **Sequence** | **SEQ ID NO:** |
|---|---|---|
| B31-CDR1 | SIMF | 6 |
| B31-CDR2 | HITSNGRTNYADSVTG | 7 |
| | HITSN**A**RTNYADSVTG | 39 |
| | HITS**Q**GRTNYADSVTG | 40 |
| B31-CDR3 | DDATNTRTY | 8 |

**Table 1B. CDR sequences of B16**

| **Name** | **Sequence** | **SEQ ID NO:** |
|---|---|---|
| B16-CDR1 | YYAIG | 9 |
| B16-CDR2 | CISSSDGSTYYADSVKG | 10 |
| | CISSSD**A**STYYADSVKG | 41 |
| | CISSS**E**GSTYYADSVKG | 42 |
| B16-CDR3 | DTTTRCPIWSYYVESDY | 11 |

**Table 1C. CDR sequences of B125**

| **Name** | **Sequence** | **SEQ ID NO:** |
|---|---|---|
| B125-CDR1 | DYAIG | 12 |
| B125-CDR2 | CISSSDGSTYYADSVKG | 13 |
| | CISSSD**A**STYYADSVKG | 43 |
| | CISSS**E**GSTYYADSVKG | 44 |
| B125-CDR3 | DTTTRCPIWSYYVESDY | 14 |

**Table 1D. CDR sequences of B164**

| **Name** | **Sequence** | **SEQ ID NO:** |
|---|---|---|
| B164-CDR1 | SYMF | 15 |
| B164-CDR2 | HITSGGRTNYADSVTG | 16 |
| B164-CDR3 | DDAVYTDRTY | 17 |

**Table 1E. CDR sequences of B210**

| **Name** | **Sequence** | **SEQ ID NO:** |
|---|---|---|
| B210-CDR1 | YYAIG | 18 |
| B210-CDR2 | CISSSGRSTNYADSVKG | 19 |
| B210-CDR3 | DTTTRCPIWSNYVESDY | 20 |

### Example 2: Binding activity to 4-1BB antigen

This example tested the binding activities of the antibodies to the 4-1BB protein.

### 2.1 ELISA binding to 4-1BB

To evaluate the binding activity of clones B31, B16, B125, B164, and B210, the chimeric mAb from these clones were subjected to ELISA test, along with Urelumab, a reference anti-4-1BB agonist antibody.

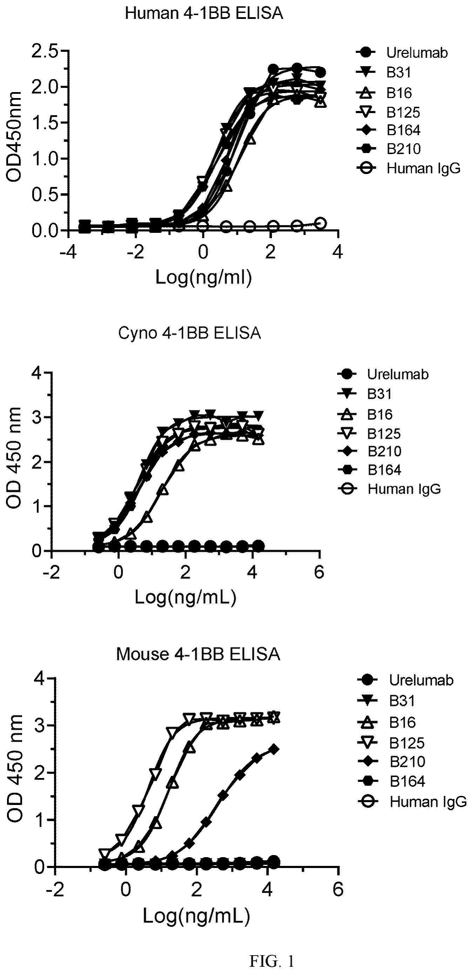

Briefly, microtiter plates were coated with human 4-1BB-His protein at 0.5 µg/ml in PBS, 100µl/well at 4°C overnight, then blocked with 150µl/well of 1% BSA. Three-fold dilutions of B31, B16, B125, B164, and B210 antibodies starting from 3 µg/ml were added to each well and incubated for 1 hour at 37°C. The plates were washed with PBS/Tween and then incubated with Anti-Human IgG (H&L) (GOAT) Antibody Peroxidase Conjugated for 30 mins at 37°C. After washing, the plates were developed with TMB substrate and analyzed by spectrophotometer at OD 450nm. As shown in **FIG. 1** and **Table 2,** all these clones bound to human 4-1BB at high affinity.

Microtiter plates were coated with cynomolgus 4-1BB-His protein at 0.5 µg/ml in PBS, 100µl/well at 4°C overnight, then blocked with 150µl/well of 1% BSA. Three-fold dilutions of B31, B16, B125, B164, and B210 antibodies starting from 15 µg/ml were added to each well and incubated for 1 hour at 37°C. The plates were washed with PBS/Tween and then incubated with Anti-Human IgG (H&L) (GOAT) Antibody Peroxidase Conjugated for 30 mins at 37°C. After washing, the plates were developed with TMB substrate and analyzed by spectrophotometer at OD 450nm. As shown in **FIG. 1** and **Table 2,** all these clones bound to cynomolgus 4-1BB at high affinity.

Microtiter plates were coated with mouse 4-1BB-His protein at 0.5 µg/ml in PBS, 100µl/well at 4°C overnight, then blocked with 150µl/well of 1% BSA. Three-fold dilutions of B31, B16, B125, B164, and B210 antibodies starting from 15 µg/ml were added to each well and incubated for 1 hour at 37°C. The plates were washed with PBS/Tween and then incubated with Anti-Human IgG (H&L) (GOAT) Antibody Peroxidase Conjugated for 30 mins at 37°C. After washing, the plates were developed with TMB substrate and analyzed by spectrophotometer at OD 450nm. As shown in **FIG. 1** and **Table 2,** only B16, B125, B210 bound to mouse 4-1BB.

**Table 2. Cross species activity of B31, B16, B125, B164, B210**

| | **Human** | **Cynomolgus** | **Mouse** |
|---|---|---|---|
| EC50 of B31 | 2.415 ng/ml | 4.063 ng/ml | -- |
| EC50 of B16 | 10.6 ng/ml | 19.52 ng/ml | 16.29 ng/ml |
| EC50 of B125 | 2.055 ng/ml | 3.543 ng/ml | 4.424 ng/ml |
| EC50 of B164 | 5.197 ng/ml | 4.808 ng/ml | - |
| EC50 of B210 | 2.514 ng/ml | 3.911 ng/ml | 401.8 ng/ml |

| | | | |
|---|---|---|---|
| --: No binding | | | |

### 2.2 Cell-based binding to 4-1BB

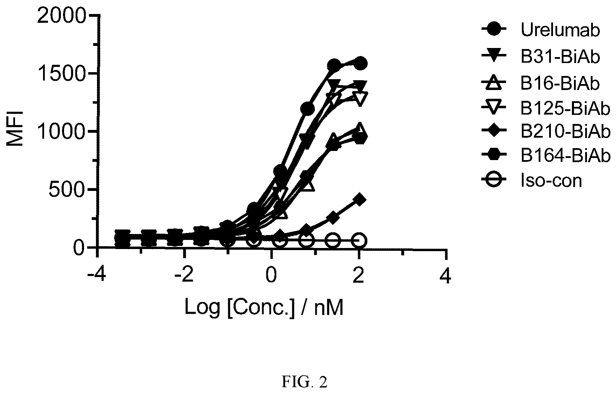

To evaluate the 4-1BB binding property, the anti-CLDN18.2-4-1BB bispecific antibodies were analyzed for their binding to HEK293 expressed 4-1BB by FACS. A total number of 1X10⁵ HEK293-4-1BB cells in each well were incubated with 4-fold serial diluted antibodies starting from 100 nM for 30 minutes at 4°C in FACS buffer. After wash by FACS buffer, PE conjugated-anti-human IgG antibody was added to each well and incubated at 4°C for 30 minutes. After wash, MFI of PE was evaluated by MACSQuant Analyzer 16. As shown in **FIG. 2****,** the tested anti-CLDN18.2-4-1BB bispecific antibodies showed concentration-dependent binding abilities to 4-1BB.

### 2.3 Protein full kinetic for 4-1BB

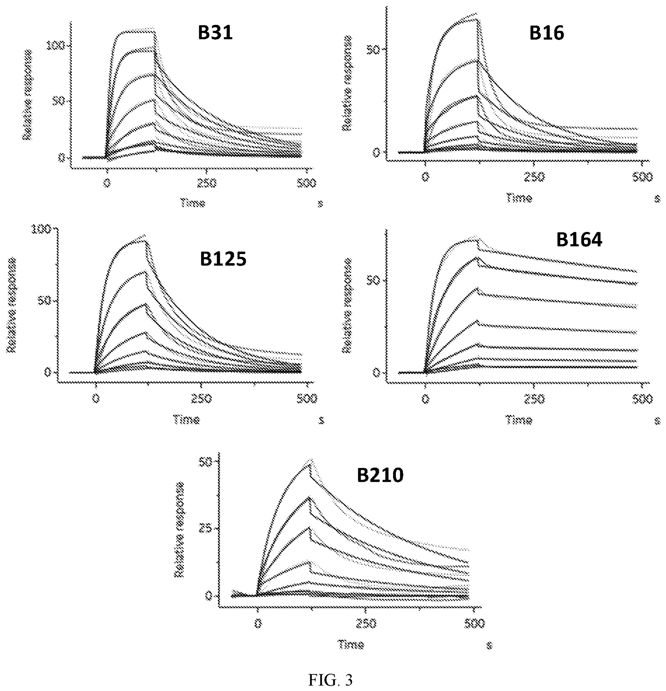

The binding of the B31, B16, B125, B164, and B210 antibodies to recombinant 4-1BB protein (human 4-1BB-his tag) was tested with Biacore using a capture method. The B31, B16, B125, B164, and B210 mAbs were captured using Protein A chip. A serial dilution of human 4-1BB-his tag protein was injected over captured antibody for 30s at a flow rate of 30 µl/min. The antigen was allowed to dissociate for 360s. All the experiments were carried out on a Biacore T200. Data analysis was carried out using the Biacore T200 evaluation software. The results are shown in **FIG.3** and **Table 3** below.

**Table 3: Full kinetic measured by Biacore**

| **Abs** | **Human 4-1BB-His** | | |
|---|---|---|---|
| | **ka (1/Ms)** | **kd (1/s)** | **KD (M)** |
| **B31** | 1.52E+06 | 4.80E-03 | 3.16E-09 |
| **B16** | 3.43E+05 | 7.39E-03 | 2.15E-08 |
| **B125** | 3.90E+05 | 8.18E-03 | 2.10E-08 |
| **B164** | 1.04E+06 | 5.46E-04 | 5.26E-10 |
| **B210** | 3.89E+04 | 3.52E-03 | 9.04E-08 |

### 2.4 Cross binding activity with OX40 and CD40

To evaluate the cross-binding activity of clones B31, B16, B125, B164, and B210 with human CD40 and human OX40, the chimeric mAb from these clones were subjected to ELISA testing.

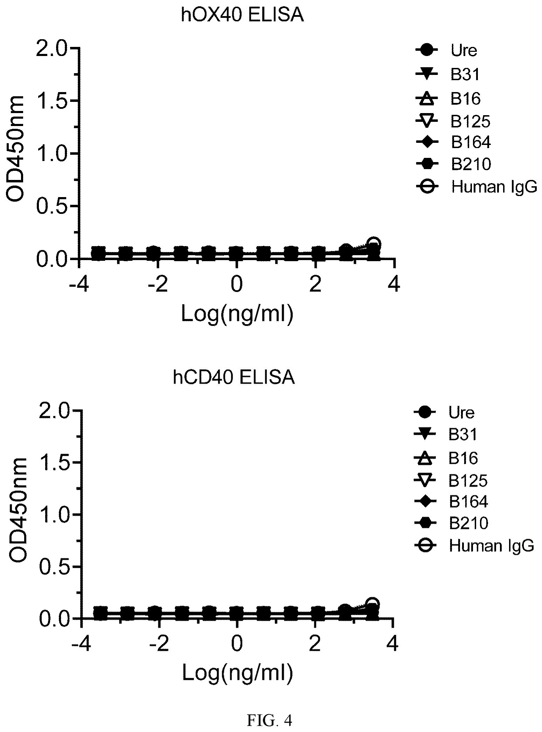

Briefly, microtiter plates were coated with human CD40 protein or human OX40 protein at 0.5 µg/ml in PBS, 100 µl/well at 4°C overnight, then blocked with 150µl/well of 1% BSA. Five-fold dilutions of B31, B16, B125, B164, and B210 antibodies starting from 3 µg/ml were added to each well and incubated for 1 hour at 37°C. The plates were washed with PBS/Tween and then incubated with Anti-Human IgG (H&L) (GOAT) Antibody Peroxidase Conjugated for 30 mins at 37°C. After washing, the plates were developed with TMB substrate and analyzed by spectrophotometer at OD 450nm. As shown in **FIG. 4****,** all these clones do not cross-bound to human CD40 or human OX40.

### Example 3. Functional Activity of 4-1BB Nanobodies

This example tested the functional activities of the antibodies, and shows that unlike Urelumab, the instant antibodies did not activate 4-1BB signaling.

### Cell-line based functional characterization of 4-IBB monoclonal antibodies

To assess the ability of the 4-1BB monoclonal antibodies to activate the 4-1BB signaling pathway, a commercial 4-1BB NF-κB luciferase reporter system was used. In this assay, HEK-4-1BB NF-κB is used as reporter cell line. HEK-4-1BB NF-κB cell line is genetically modified to stably express 4-1BB and luciferase downstream of a response element (Genomeditech, cat# GM-C04832). Luciferase expression is induced upon antibody binding to the 4-1BB receptor. Briefly, reporter cells at a density of 2.5X10⁴ cells per well were cultured in a white 96-well plate. Antibodies were serially diluted and added to a white 96-well assay plate, at a final concentration ranging from 150 nM to 0.0000768 nM for B31 molecule and 100 nM to 0.000381 nM for B16, B125, B164, and B210 molecules. Following a 6 hours incubation at 37°C, luminescence was obtained by adding the substrate of luciferase and measured by a microplate reader. Four-parameter logistic curve analysis was performed with GraphPad software.

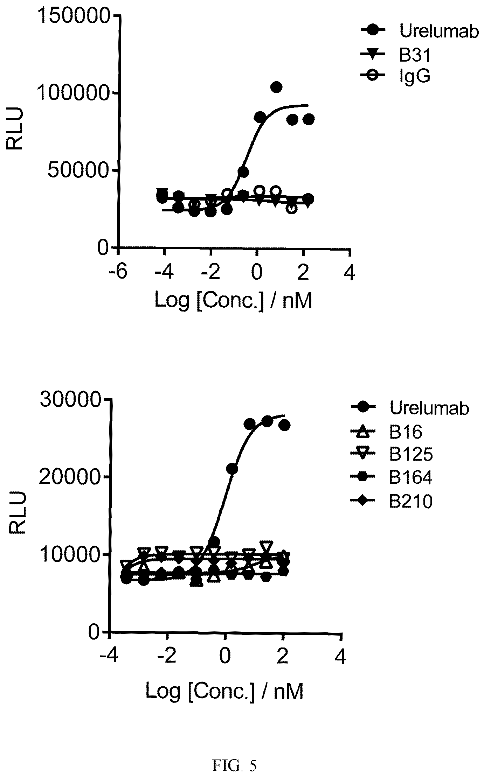

As shown in **FIG. 5****,** the Urelumab monoclonal antibody dose-dependently activated the 4-1BB signaling, while the B31, B16, B125, B164, and B210 antibodies did not boost 4-1BB induction in the same experimental settings.

### Example 4. Functional Activity of Anti-Claudin 18.2/4-1BB Bispecific Antibodies

In this example, bispecific antibodies that incorporate the anti-4-1BB nanobodies and also have a specificity to Claudin 18.2 were generated and tested.

### 4.1 Cell-line based functional characterization of Claudin 18.2-4-1BB bispecific antibody

To assess the ability of the generated anti-Claudin 18.2/4-1BB bispecific antibodies to activate 4-1BB signaling pathway, a commercial 4-1BB NF-κB luciferase reporter system was used. In this assay, H_TNFRSF9(4-1BB) NFκB-Reporter Jurkat (Genomeditech, cat# GM-C09468) was used as effector cells and CHO-Kl-expressing or not expressing Claudin 18.2 as target cells. H_TNFRSF9(4-1BB) NFKB-Reporter Jurkat cell line is genetically modified to stably express 4-1BB and luciferase downstream of a response element. Luciferase expression is induced upon antibody binding to the 4-1BB receptor. Briefly, effector cells at a density of 2.5X10⁴ cells per well were cocultured with 2.5X10⁴ target cells (E/T Ratio=1:1) in a white 96-well plate. Antibodies were 4-fold serially diluted and added to a white 96-well assay plate, at a final concentration ranging from 100 nM to 0.000381 nM. Following a 6 hours incubation at 37°C, luminescence was obtained by adding the substrate of luciferase and measured by a microplate reader. Four-parameter logistic curve analysis was performed with GraphPad software.

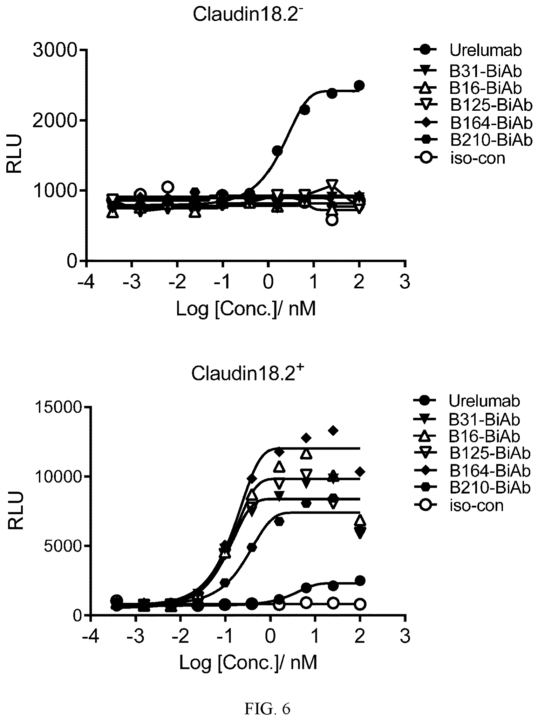

As shown in **FIG. 6****,** the Urelumab monoclonal antibody can dose-dependently boost the 4-1BB signaling in both CHO-K1 and CHO-Claudin 18.2 overexpression cells. While the activity of anti-Claudin18.2/4-1BB bispecific antibodies B31-BiAb, B16-BiAb, B125-BiAb, B164-BiAb and B210-BiAb were dependent on the expression of Claudin 18.2 on the cells.

### 4.2 Activity of the bispecific antibodies to promote human peripheral blood mononuclear cell (PBMC) immune response

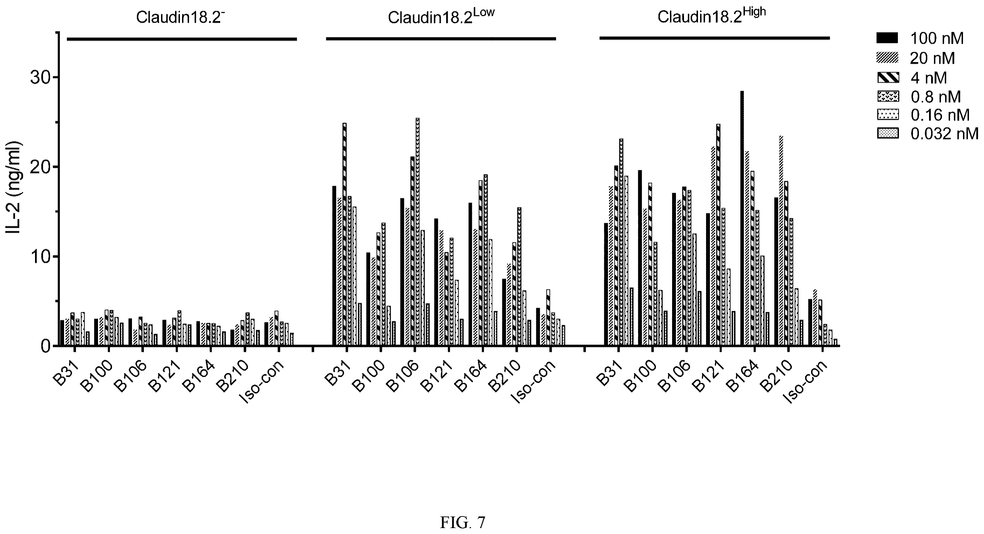

To investigate the ability of the Claudin 18.2-4-1BB bispecific antibodies to stimulated human PBMCs response, IL-2 cytokine release was examined by LANCE Ultra TR-FRET Detection Kit. Human PBMCs stimulated with 0.5 µg/ml human anti-CD3 antibody were used as the effector cells. CHO-K1 expressing Claudin 18.2 was used as the target cells. Human PBMCs (1×10⁵) were co-cultured with CHO-K1-Claudin 18.2 or parental CHO-K1 cells (2.5×10⁴) (E/T Ratio=4:1) in the presence of human anti-CD3 antibody. 5-fold serially diluted bispecific antibodies were added to the culture medium at a final concentration starting from 100 nM. 48 hours later, the secretion level of IL2 in the culture medium was measured using IL-2 (human) LANCE Ultra TR-FRET Detection Kit (PerkinElmer). As shown in **FIG. 7****,** the bispecific antibodies could only activate PBMC response in the presence of Claudin 18.2 over-expressing cells.

### Example 5. Humanization of the B31/B125/B16/B210 VHH Antibodies

The B31/B125/B16/B210 variable region genes were employed to create a humanized mAb. In the first step of this process, the amino acid sequences of the VHH of B31/B125/B16/B210 were compared against the available database of human Ig gene sequences to find the overall best-matching human germline Ig gene sequences. Humanized variable domain sequences of B31/B125/B16/B210 were then designed where the CDRH1, H2, and H3 onto framework sequences of the VH gene.

The amino acid and nucleotide sequences of some of the humanized antibody are listed in **Table 4** below.

**Table 4A. Humanized antibody sequences of B31 (underlining indicates CDR; bold/italic indicates back mutations)**

| **B31** | **Sequence** | **SEQ ID NO:** |
|---|---|---|
| VHH | QVQLVESGGGLVQAGGSLRLSCAASGDIFSIMFWYRQPQGKQREYVA HITSNGRTNYADSVTGRFTISRGNNDNTMYLQMDNLKPQDTAVYYCA ADDATNTRTYWGQGTQVTVSS | 1 |
| VHH-V2 | QVQLVESGGGLVQPGGSLRLSCSASGDIFSIMFWYRQAPGKQREYVS HITSNGRTNYADSVTGRFTISRDNSKNTLYLQMNSLRAEDTAVYYCA ADDATNTRTYWGQGTLVTVSS | 21 |
| VHH-V3 | QVQLVESGGGLVQPGGSLRLSCAASGDIFSIMFWYRQAPGKQREYVA HITSNGRTNYADSVTGRFTISRDNNKNTLYLQMNSLRAEDTAVYYCA ADDATNTRTYWGQGTLVTVSS | 22 |
| VHH-V4 | QVQLVESGGGLVQPGGSLRLSCAASGDIFSIMFWYRQAPGKQREYVA HITSNGRTNYADSVTGRFTISRDNSKNTMYLQMDSLRAEDTAVYYCA ADDATNTRTYWGQGTLVTVSS | 23 |
| VHH-V5 | QVQLVESGGGLVQPGGSLRLSCAASGDIFSIMFWYRQPQGKQREYVA HITSNGRTNYADSVTGRFTISRDNNKNTLYLQMDSLRAEDTAVYYCA ADDATNTRTYWGQGTLVTVSS | 24 |
| VHH-V6 | QVQLVESGGGLVQPGGSLRLSCSASGDIFSIMFWYRQAPGKQREYVA HITSNGRTNYADSVTGRFTISRDNSKNTLYLQMNSLRAEDTAVYYCA RDDATNTRTYWGQGTLVTVSS | 25 |

**Table 4B. Humanized antibody sequences of B125 (underlining indicates CDR; bold/italic indicates back mutations)**

| **B125** | **Sequence** | **SEQ ID NO:** |
|---|---|---|
| VHH | QVQLVESGGGLVQPGGSLRLSCAASGFAFSDYAIGWFRQAPGKEREG VSCISSSDGSTYYADSVKGRFTISRDNAKNTVYLQMNSLKPEDTAVY YCATDTTTRCPIWSYYVESDYWGQGTQVTVSS | 3 |
| VHH-V1 | EVQLVESGGGLVQPGGSLRLSCAASGFAFSDYAIGWVRQAPGKGLEW VSCISSSDGSTYYADSVKGRFTISRDNSKNTLYLQMNSLRAEDTAVY YCAKDTTTRCPIWSYYVESDYWGQGTLVTVSS | 26 |
| VHH-V2 | EVQLVESGGGLVQPGGSLRLSCAASGFAFSDYAIGWFRQAPGKEREG VSCISSSDGSTYYADSVKGRFTISRDNSKNTLYLQMNSLRAEDTAVY YCATDTTTRCPIWSYYVESDYWGQGTLVTVSS | 27 |
| VHH-V3 | EVQLVESGGGLVQPGGSLRLSCAASGFAFSDYAIGWFRQAPGKEREG VSCISSSDGSTYYADSVKGRFTISRDNAKNTLYLQMNSLRAEDTAVY YCAKDTTTRCPIWSYYVESDYWGQGTLVTVSS | 28 |
| VHH-V4 | EVQLVESGGGLVQPGGSLRLSCAASGFAFSDYAIGWFRQAPGKEREG VSCISSSDGSTYYADSVKGRFTISRDNAKNTLYLQMNSLRAEDTAVY YCATDTTTRCPIWSYYVESDYWGQGTLVTVSS | 29 |
| VHH-V5 | EVQLVESGGGLVQPGGSLRLSCAASGFAFSDYAIGWFRQAPGKEREG VSCISSSDGSTYYADSVKGRFTISRDNAKNTLYLQMNSLRPEDTAVY YCATDTTTRCPIWSYYVESDYWGQGTLVTVSS | 30 |

**Table 4C. Humanized antibody sequences of B16 (underlining indicates CDR; bold/italic indicates back mutations)**

| **B16** | **Sequence** | **SEQ ID NO:** |
|---|---|---|
| VHH | QVQLVESGGGLVQPGGSLRLSCAASGFTLDYYAIGWFRQAPGKEREG VSCISSSDGSTYYADSVKGRFTISRDNAKNTVYLQMNSLKPEDTAVY YCATDTTTRCPIWSYYVESDYWGQGTQVTVSS | 2 |
| VHH-V1 | EVQLVESGGGLVQPGGSLRLSCAASGFTFSYYAIGWVRQAPGKGLEW VSCISSSDGSTYYADSVKGRFTISRDNSKNTLYLQMNSLRAEDTAVY YCAKDTTTRCPIWSYYVESDYWGQGTLVTVSS | 31 |
| VHH-V2 | EVQLVESGGGLVQPGGSLRLSCAASGFTFSYYAIGWFRQAPGKEREG VSCISSSDGSTYYADSVKGRFTISRDNSKNTLYLQMNSLRAEDTAVY YCATDTTTRCPIWSYYVESDYWGQGTLVTVSS | 32 |
| VHH-V3 | EVQLVESGGGLVQPGGSLRLSCAASGFTLDYYAIGWFRQAPGKEREG VSCISSSDGSTYYADSVKGRFTISRDNSKNTLYLQMNSLRAEDTAVY YCATDTTTRCPIWSYYVESDYWGQGTLVTVSS | 33 |
| VHH-V4 | EVQLVESGGGLVQPGGSLRLSCAASGFTLDYYAIGWFRQAPGKEREG VSCISSSDGSTYYADSVKGRFTISRDNAKNTLYLQMNSLRPEDTAVY YCATDTTTRCPIWSYYVESDYWGQGTLVTVSS | 34 |

**Table 4D. Humanized antibody sequences of B210 (underlining indicates CDR; bold/italic indicates back mutations)**

| **B210** | **Sequence** | **SEQ ID NO:** |
|---|---|---|
| VHH | EVQLVESGGGLVQPGGSLRLSCAASGLSLDYYAIGWFRQAPGKEREG VSCISSSGRSTNYADSVKGRFTISRDNAKNTVYLQMNSLKPEDTADY YCATDTTTRCPIWSNYVESDYWGQGTQVTVSS | 5 |
| VHH-V1 | EVQLVESGGGLVQPGGSLRLSCAASGFTFSYYAIGWVRQAPGKGLEW VSCISSSGRSTNYADSVKGRFTISRDNSKNTLYLQMNSLRAEDTAVY YCAKDTTTRCPIWSNYVESDYWGQGTLVTVSS | 35 |
| VHH-V2 | EVQLVESGGGLVQPGGSLRLSCAASGFTFSYYAIGWFRQAPGKEREG VSCISSSGRSTNYADSVKGRFTISRDNSKNTLYLQMNSLRAEDTAVY YCATDTTTRCPIWSNYVESDYWGQGTLVTVSS | 36 |
| VHH-V3 | EVQLVESGGGLVQPGGSLRLSCAASGLTFSYYAIGWFRQAPGKEREG VSCISSSGRSTNYADSVKGRFTISRDNSKNTLYLQMNSLRAEDTAVY YCATDTTTRCPIWSNYVESDYWGQGTLVTVSS | 37 |
| VHH-V4 | EVQLVESGGGLVQPGGSLRLSCAASGLTLSYYAIGWFRQAPGKEREG VSCISSSGRSTNYADSVKGRFTISRDNSKNTLYLQMNSLRAEDTADY YCATDTTTRCPIWSNYVESDYWGQGTLVTVSS | 38 |

The back mutations of B31 include 23A, 37Y, 40P, 41Q, 44Q, 45R, 49A, 74N, 78M, 82(82A)D, and 94A. More specifically, VHH-v2 included back mutations 37Y, 44Q, 45R and 94A; VHH-v3 included back mutations 23A, 37Y, 44Q, 45R, 49A, 74N and 94A; VHH-v4 included back mutations 23A, 37Y, 44Q, 45R, 49A, 78M, 82(82A)D, and 94A; VHH-v5 included back mutations 23A, 37Y, 40P, 41Q, 44Q, 45R, 49A, 74N, 82(82A)D, and 94A; and VHH-v6 included back mutations 37Y, 44Q, 45R, and 49A.

The back mutations of B16 include 29L, 30D, 37F, 44E, 45R, 47G, 74A, 84P, and 94T. More specifically, VHH-v2 included back mutations 37F, 44E, 45R, 47G, and 94T; VHH-v3 included back mutations 29L, 30D, 37F, 44E, 45R, 47G and 94T; VHH-v4 included back mutations 29L, 30D, 37F, 44E, 45R, 47G, 74A, 84P, and 94T.

The back mutations of B125 include 37F, 43K, 44E, 45R, 47G, 74A, 84P, and 94T. More specifically, VHH-v2 included back mutations 37F, 44E, 45R, 47G, and 94T; VHH-v3 included back mutations 37F, 43K, 44E, 45R, 47G and 74A; VHH-v4 included back mutations 37F, 43K, 44E, 45R, 47G, 74A, and 94T; VHH-v5 included back mutations 37F, 43K, 44E, 45R, 47G, 74A, 84P, and 94T.

The back mutations of B210 include 27L, 29L, 37F, 44E, 45R, 47G, 89D, and 94T. More specifically, VHH-v2 included back mutations 37F, 44E, 45R, 47G, and 94T; VHH-v3 included back mutations 27L, 37F, 44E, 45R, 47G and 94T; VHH-v4 included back mutations 27L, 29L, 37F, 44E, 45R, 47G and 94T; VHH-v5 included back mutations 27L, 29L, 37F, 44E, 45R, 47G, 89D, and 94T.

The humanized VHH genes were cloned into pcDNA3.4 vectors and transfected into 293F cells for further analysis.

### Example 6: Antigen binding properties of the Humanized Antibodies

### Full kinetic affinity of humanized antibodies by Biacore

The binding of the humanized antibodies to recombinant human 4-1BB protein (human 4-1BB-his tag) was tested by Biacore using a capture method. B31-V2, B31-V3, B31-V4, B31-V5, and B31-V6 were captured using Protein A chip. A serial dilution of human 4-1BB-his tag protein was injected over captured antibody for 30s at a flow rate of 10 µl/min. The antigen was allowed to dissociate for 360s. All the experiments were carried out on a Biacore T200. Data analysis was carried out using Biacore T200 evaluation software and the results are shown in **Table 5** below. All humanized antibodies exhibited strong bindings.

**Table 5 Full kinetic by Biacore**

| **Abs** | **4-1BB-His** | | |
|---|---|---|---|
| | **ka (1/Ms)** | **kd (1/s)** | **KD (M)** |
| **B31-V2** | 5.17E+05 | 6.80E-03 | 1.31E-08 |
| **B31-V3** | 5.26E+05 | 7.16E-03 | 1.36E-08 |
| **B31-V4** | 1.15E+06 | 5.45E-03 | 4.74E-09 |
| **B31-VS** | 1.30E+06 | 6.46E-03 | 4.97E-09 |
| **B31-V6** | 2.37E+05 | 1.56E-03 | 6.58E-09 |
| **B125-V2** | 8.67E+03 | 2.66E-02 | 3.07E-06 |
| **B16-V2** | 4.65E+04 | 8.98E-02 | 1.93E-06 |
| **B210-V2** | 5.37E+04 | 4.10E-02 | 7.65E-07 |
| **B210-V3** | 1.59E+04 | 4.08E-02 | 2.56E-06 |

### Example 7. Functional Activity of humanized bispecific antibodies

Anti-Claudin 18.2/4-1BB bispecific antibodies were prepared with the humanized 4-1BB nanobodies and tested in this example.

### 7.1 Cell-line based functional characterization of Claudin 18.2-4-1BB humanized bispecific antibodies

To assess the ability of the Claudin 18.2-4-1BB humanized bispecific antibodies to activate 4-1BB signaling pathway, a commercial 4-1BB NF-κB luciferase reporter system was used. In this assay, H_TNFRSF9(4-1BB) NFκB-Reporter Jurkat (Genomeditech, cat# GM-C09468) was used as effector cells and CHO-Kl-expressing or not expressing Claudin18.2 as target cells. H_TNFRSF9(4-1BB) NFKB-Reporter Jurkat cell line is genetically modified to stably express 4-1BB and luciferase downstream of a response element. Luciferase expression is induced upon antibody binding to the 4-1BB receptor. In brief, effector cells at a density of 2.5X10⁴ cells per well were cocultured with 2.5X10⁴ target cells (E/T Ratio=1:1) in a white 96-well plate. Antibodies were 3-fold serially diluted and added to a white 96-well assay plate, at a final concentration ranging from 100 nM to 0.005 nM. Following a 6 hours incubation at 37°C, luminescence was obtained by adding the substrate of luciferase and measured by a microplate reader. Four-parameter logistic curve analysis was performed with GraphPad software.

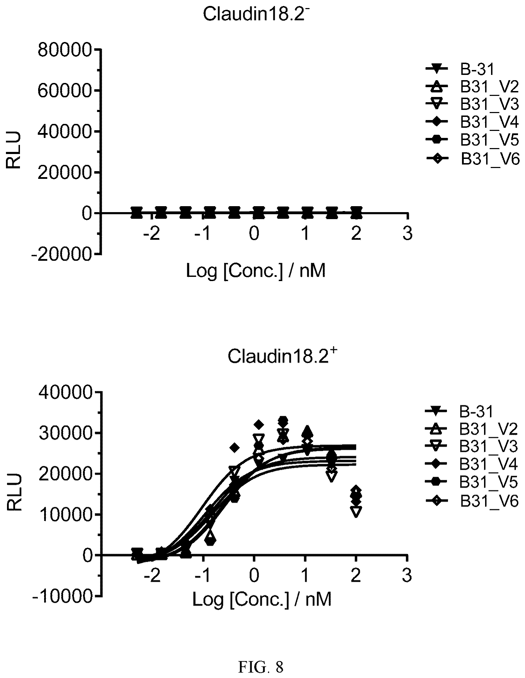

As shown in **FIG. 8****,** the activity of anti-Claudin 18.2/4-1BB humanized bispecific antibodies were comparable with the B31 chimeric antibody.

### 7.2 Activity of the humanized bispecific antibodies to promote human peripheral blood mononuclear cell (PBMC) immune response

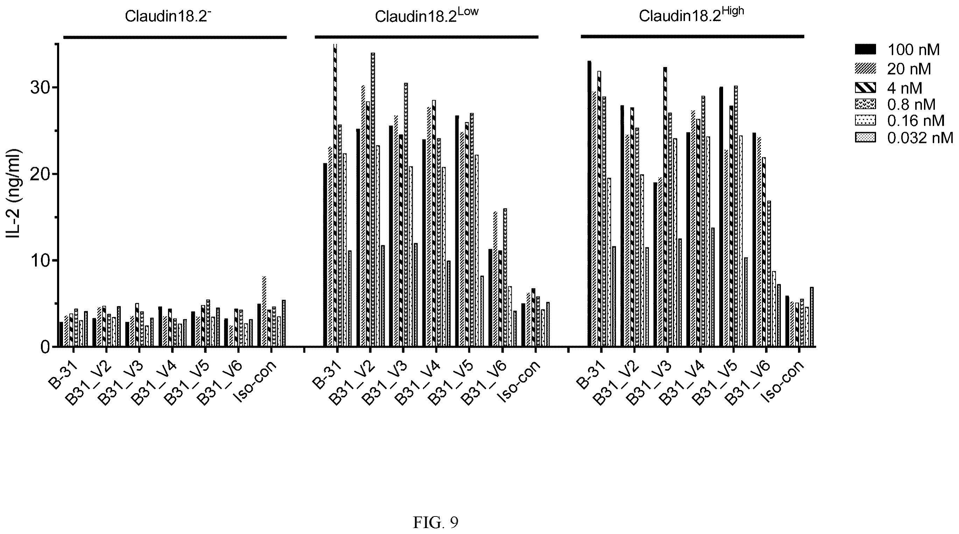

To investigate the ability of the Claudin 18.2-4-1BB humanized bispecific antibodies to stimulated human PBMCs response, IL-2 cytokine release was examined by LANCE Ultra TR-FRET Detection Kit. Human PBMCs stimulated with 0.5 µg/ml human anti-CD3 antibody were used as the effector cells. CHO-K1 express Claudin 18.2 was used as the target cells. Human PBMCs (1×10⁵) were co-cultured with CHO-K1-Claudin 18.2 or parental CHO-K1 cells (2.5×10⁴) (E/T Ratio=4:1) in the presence of human anti-CD3 antibody. 5-fold serially diluted humanized bispecific antibodies were added to the culture medium at a final concentration starting from 100 nM. 48 hours later, the secretion level of IL2 in the culture medium was measured using IL-2 (human) LANCE Ultra TR-FRET Detection Kit (PerkinElmer). As shown in **FIG. 9****,** the IL-2 secretion induced by humanized bispecific antibodies were comparable with the B31 chimeric antibody.

### Example 8. Tumor Growth Inhibition by Anti-Claudin 18.2/4-1BB Bispecific Antibodies

In this example, humanized mice that expressed the extracellular domain of human 4-1BB were used to test the activity of the bispecific antibodies to inhibit tumor growth.

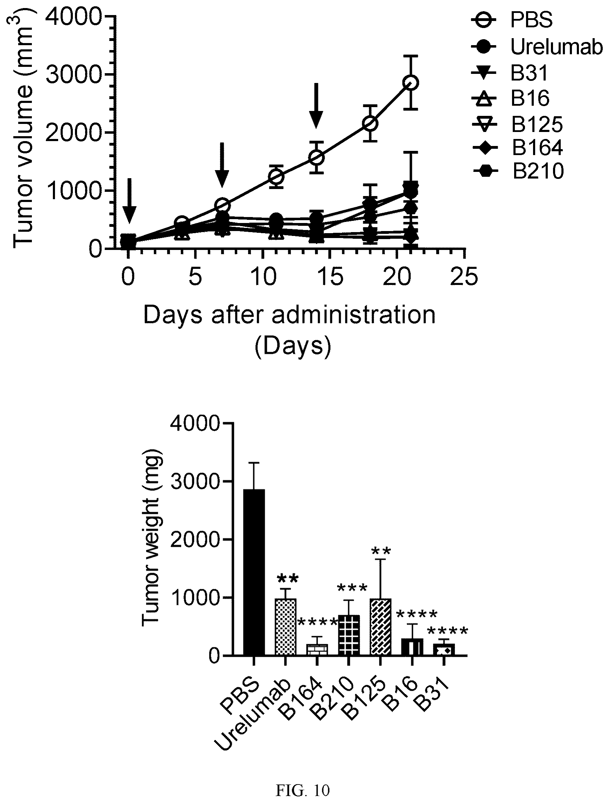

Mouse colon adenocarcinoma cells (MC38) were engineered to express human CLDN18.2. Humanized mice (h4-1BB) were subcutaneously implanted with MC38-hCLDN18.2 cells. Mouse were intraperitoneally administered every week for 3 times with following antibodies: PBS control (1 mg/kg), Urelumab (0.85 mg/kg), B31 anti-CLDN18.2/4-1BB bispecific antibody (1 mg/kg), B16 anti-CLDN18.2/4-1BB bispecific antibody (1 mg/kg), B125 anti/CLDN18.2-4-1BB bispecific antibody (1 mg/kg), B164 anti-CLDN18.2/4-1BB bispecific antibody (1 mg/kg), B210 anti-CLDN18.2/4-1BB bispecific antibody (1 mg/kg). Tumor volumes were monitored by caliper measurement twice per week for the duration of the experiment. As shown in **FIG. 10****,** Urelumab, B31, B16, B125, B164, and B210 all can suppress the tumor growth with the TG1 from 62.4%-96.9%.

| **Group** | **TGI (%)** |
|---|---|
| Urelumab | 68.5% |
| B31 | 96.9% |
| B16 | 93.6% |
| B125 | 68.5% |
| B164 | 97.1% |
| B210 | 78.9% |

The present disclosure is not to be limited in scope by the specific embodiments described which are intended as single illustrations of individual aspects of the disclosure, and any compositions or methods which are functionally equivalent are within the scope of this disclosure. It will be apparent to those skilled in the art that various modifications and variations can be made in the methods and compositions of the present disclosure without departing from the spirit or scope of the disclosure. Thus, it is intended that the present disclosure cover the modifications and variations of this disclosure provided they come within the scope of the appended claims and their equivalents.

All publications and patent applications mentioned in this specification are herein incorporated by reference to the same extent as if each individual publication or patent application was specifically and individually indicated to be incorporated by reference.

## Claims

1. A single domain antibody or a polypeptide comprising the single domain antibody, wherein the single domain antibody has binding specificity to the human 4-1BB protein and comprises a complementarity determining region 1 (CDR1), a CDR2 and a CDR3, wherein:
(a) the CDR1 comprises the amino acid sequence of SEQ ID NO:6, the CDR2 comprises the amino acid sequence of SEQ ID NO:7, 39, or 40, and the CDR3 comprises the amino acid sequence of SEQ ID NO:8;
(b) the CDR1 comprises the amino acid sequence of SEQ ID NO:9, the CDR2 comprises the amino acid sequence of SEQ ID NO:10, 41 or 42, and the CDR3 comprises the amino acid sequence of SEQ ID NO:11; or
(c) the CDR1 comprises the amino acid sequence of SEQ ID NO:12, the CDR2 comprises the amino acid sequence of SEQ ID NO:13, 43 or 44, and the CDR3 comprises the amino acid sequence of SEQ ID NO:14.

2. The antibody or polypeptide of claim 1, wherein the CDR1 comprises the amino acid sequence of SEQ ID NO:6, the CDR2 comprises the amino acid sequence of SEQ ID NO:7, 39 or 40, and the CDR3 comprises the amino acid sequence of SEQ ID NO:8.

3. The antibody or polypeptide of claim 2, which comprises an amino acid sequence selected from the group consisting of SEQ ID NO:1 and 21-25.

4. The antibody or polypeptide of claim 1, wherein the CDR1 comprises the amino acid sequence of SEQ ID NO:9, the CDR2 comprises the amino acid sequence of SEQ ID NO:10, 41 or 42, and the CDR3 comprises the amino acid sequence of SEQ ID NO:11.

5. The antibody or polypeptide of claim 4, which comprises an amino acid sequence selected from the group consisting of SEQ ID NO:1 and 31-34.

6. The antibody or polypeptide of claim 1, wherein the CDR1 comprises the amino acid sequence of SEQ ID NO:12, the CDR2 comprises the amino acid sequence of SEQ ID NO:13, 43 or 44, and the CDR3 comprises the amino acid sequence of SEQ ID NO:14.

7. The antibody or polypeptide of claim 6, which comprises an amino acid sequence selected from the group consisting of SEQ ID NO:3 and 26-30.

8. The antibody or polypeptide of any one of claims 1-7, wherein the polypeptide is a chimeric antigen receptor (CAR) or a bispecific antibody having a binding specificity to an antigen different from 4-1BB.

9. A bispecific antibody comprising the antibody of any one of claims 1-7 and a second antibody or antigen-binding fragment having binding specificity to a target antigen that is not 4-1BB.

10. The bispecific antibody of claim 9, wherein the target antigen is selected from the group consisting of Claudin 18.2, EGFR, Her2, EpCAM, CD20, CD30, CD33, CD47, CD52, CD133, CD73, CEA, gpA33, Mucins, TAG-72, CIX, PSMA, folate-binding protein, GD2, GD3, GM2, VEGF, VEGFR, Integrin, αVβ3, α5β1, ERBB2, ERBB3, MET, IGF1R, EPHA3, TRAILR1, TRAILR2, RANKL, FAP and Tenascin.

11. A polynucleotide encoding the antibody or polypeptide of any one of claims 1-10.

12. A cell comprising the polynucleotide of claim 11.

13. A pharmaceutical composition for treating cancer comprising the antibody or polypeptide of any one of claims 1-10 and a pharmaceutically acceptable carrier.

14. A method of treating cancer in a patient in need thereof, comprising administering to the patient an effective amount of the antibody or polypeptide of any one of claims 1-10.

15. Use of the antibody or polypeptide of any one of claims 1-10 for the preparation of a medicament for treating cancer.
